# EUROPÄISCHE PATENTSCHRIFT

(11) **EP 0 497 121 B1**
(45) Veröffentlichungstag und Bekanntmachung des Hinweises auf die Patenterteilung: **26.02.1997**
(21) Anmeldenummer: 92100380.2
(22) Anmeldetag: 11.01.1992
(51) Int. Cl.: C07D 413/14, A61K 31/415

(54) **Neue Tetrazolderivate, Verfahren zu ihrer Herstellung und ihre Verwendung**
Tetrazole derivatives, process for their production and their application
Dérivés de tétrazole, procédé pour leur fabrication et leur application

(30) Priorität: 31.01.1991 AT 209/91
(43) Veröffentlichungstag der Anmeldung: 05.08.1992
(73) Patentinhaber: Chemisch Pharmazeutische Forschungsgesellschaft m.b.H., A-4021 Linz (AT)
(72) Erfinder: Binder, Dieter, Dr., A-1190 Wien (AT); Weinberger, Josef, Dr., A-4540 Bad Hall (AT); Koch, Andreas, Dr., A-7210 Mattersburg (AT)
(74) Vertreter: Matthews, Derek Peter

(56) Entgegenhaltungen:
- EP-A- 0 291 969
- EP-A- 0 324 377
- EP-A- 0 401 030
- WO-A-91/00277

## Beschreibung

Die Erfindung betrifft neue therapeutisch wertvolle Tetrazolderivate, ein Verfahren zu ihrer Herstellung und ihre Verwendung.

Aus der Literatur ist bereits eine Vielzahl von Verbindungen bekannt, die zur Behandlung von Bluthochdruck, verursacht durch Angiotensin II, verwendet werden können.
Ein bekannter Angiotensin II Rezeptor Antagonist, DuP 753 (2-n-Butyl-4-chloro-5-hydroxymethyl-1-((2'-(1-H-tetrazol-5-yl)biphenyl-4-yl)methyl)imidazol), wird z.B. im Journal of Pharmacology and Experimental Therapeutics, P.C. Wong et.al. 1990, Vol. 255, Seiten 211- 217 beschrieben. DuP 753 wird jedoch bei der in vivo Anwendung zu einem nicht kompetitiven Metaboliten, EXP 3174, (2-n-Butyl-4-chloro-1-((2'-(1H-tetrazol-5-yl)biphenyl-4-yl)methyl)imidazol-5-carbonsäure), umgewandelt, der zu einem Großteil für die Wirkungsdauer von DuP 753 verantwortlich ist. Der Nachteil von nicht kompetitiven Antagonisten ist jedoch, daß sie irreversibel an den Rezeptor gebunden werden und dort Änderungen der zellulären Struktur auslösen.
Aufgabe der vorliegenden Erfindung war es somit, rein kompetitive Antagonisten, die keine nicht kompetitiven Metaboliten bilden, zu finden.
Diese Aufgabe konnte nun unerwarteterweise mit den erfindungsgemäßen Substanzen gelöst werden.

Gegenstand der Erfindung sind daher neue Tetrazolderivate der Formel 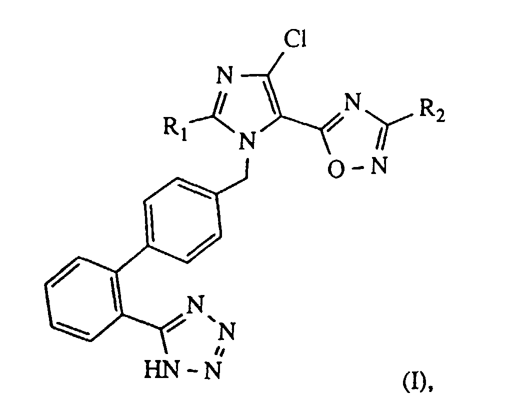 in der R₁ einen gesättigten oder ungesättigten, geradkettigen oder verzweigten (C₁ - C₆) Alkylrest und R₂ Methyl oder Ethyl bedeuten, sowie ihre pharmazeutisch verwendbaren Salze.

In der Formel I bedeutet R₁ einen gesättigten oder ungesättigten, geradkettigen oder verzweigten (C₁ - C₆) Alkylrest, wie z. B. Methyl, Ethyl, Propyl, i-Propyl, Butyl, sec. Butyl, tert. Butyl, Hexyl, Ethenyl, Propenyl, Hexenyl. Bevorzugt sind Verbindungen, in denen R₁ Butyl, sowie solche, in denen R₂ Methyl bedeutet.

Die Erfindung bezieht sich weiterhin auf ein Verfahren zur Herstellung von Verbindungen der Formel 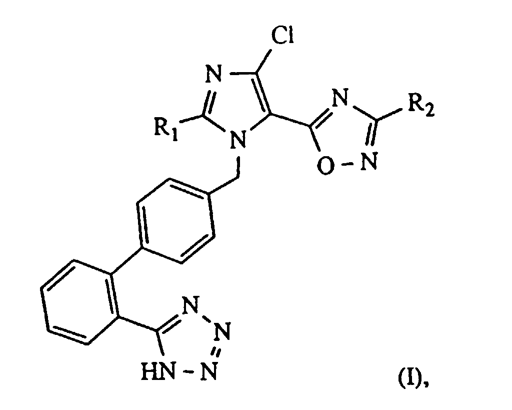 in der R₁ einen gesättigten oder ungesättigten, geradkettigen oder verzweigten (C₁ - C₆) Alkylrest und R₂ Methyl oder Ethyl bedeuten, sowie ihre pharmazeutisch verwendbaren Salze, dadurch gekennzeichnet, daß eine Verbindung der Formel 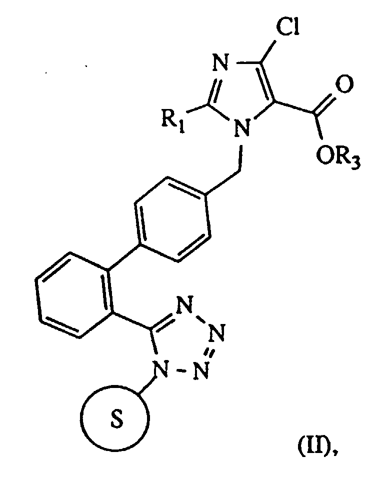 in der R₁ die obige Bedeutung hat und R₃ Methyl oder Ethyl und S eine Schutzgruppe bedeuten, mit einer Verbindung der Formel 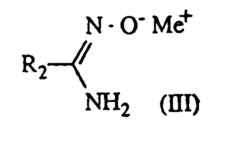 in der R₂ Methyl oder Ethyl und Me Natrium oder Kalium bedeuten, in einem unter Reaktionsbedingungen inerten organischen Verdünnungsmittel unter Inertgasatmosphäre zu einer Verbindung der Formel 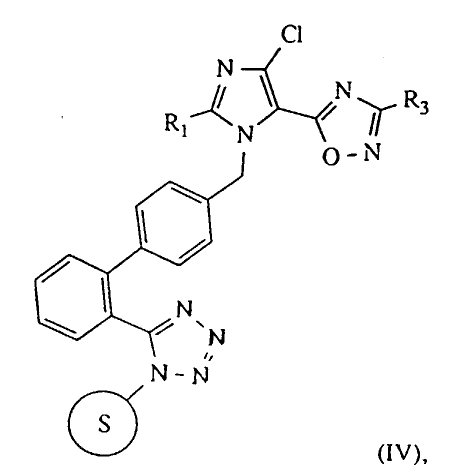 worin R₁ und R₂ die obige Bedeutung haben, umgesetzt und anschließend die Schutzgruppe entfernt wird.

Die erfindungsgemäße Umsetzung wird so durchgeführt, daß man eine Suspension der Verbindung der Formel III in einem unter Reaktionsbedingungen inerten, wasserfreien organischen Verdünnungsmittel, wie z. B. in einem Äther, wie etwa Tetrahydrofuran, Diethylether oder Dioxan unter Inertgasatmosphäre wie z. B. Argon, Helium oder Stickstoff, mit einer Verbindung der Formel II im gleichen Verdünnungsmittel gelöst, umsetzt. Die Reaktionstemperatur liegt etwa zwischen -20 und +40°C, vorzugsweise zwischen -10°C und +20°C, die Reaktionszeit beträgt abhängig von den jeweiligen Reaktionspartnern und den Reaktionsbedingungen etwa 2 bis 40 Stunden, vorzugsweise 2 bis 20 Stunden. Als Schutzgruppen werden z. B. Triphenylmethyl-, Benzyl-, p-Nitrobenzyl- oder 1-Ethoxyethylgruppen verwendet.

Die folgende Abspaltung der Schutzgruppe S aus den erhaltenen Verbindungen der Formel IV erfolgt je nach verwendeter Schutzgruppe etwa durch Rühren in einem Gemisch von HBr/Eisessig in Chloroform oder Trifluoressigsäure in Chloroform bei Raumtemperatur oder besser durch Erhitzen in einem niederen aliphatischen Alkohol, wie z. B. Methanol oder Ethanol.

Die Verbindungen der Formel II und III sind literaturbekannt (T. Hirotsu et al. J. Chem. Soc. Dalton, 1609, 1986; D.J. Carini et al. EP 0 324 377, 1989).

Die Verbindungen der Formel I können auf übliche Weise mit anorganischen und organischen Basen in ihre pharmazeutisch verwendbaren Salze übergeführt werden. Die Salzbildung kann beispielsweise durchgeführt werden, indem man die genannten Verbindungen der Formel I in einem geeigneten Mittel, z. B. Wasser, einem niederen aliphatischen Alkohol, einem Äther, wie Tetrahydrofuran, Dioxan, Diethylether; Dimethylformamid oder Dimethylsulfoxid, löst, eine äquivalente Menge der gewünschten Base zusetzt, für eine gute Durchmischung sorgt und nach beendeter Salzbildung das Lösungsmittel verdampft. Gegebenenfalls können die Salze nach der Isolierung umkristallisiert werden.

Pharmazeutisch verwendbare Salze sind z. B. Metallsalze, insbesonders Alkalimetall- oder Erdalkalimetallsalze, wie Natrium-, Kalium-, Magnesium- oder Calciumsalze. Andere pharmazeutisch verwendbare Salze sind beispielsweise leicht kristallisierende Ammoniumsalze. Letztere leiten sich von Ammoniak oder organischen Aminen, z. B. von Mono-, Di-, oder Trialkyl-, Cycloalkyl- oder Hydroxyalkyl-aminen, Alkylendiaminen oder Hydroxy-alkyl-, Arylalkyl- oder Alkylammoniumbasen, z. B. Methylamin, Diethylamin, Triethylamin, Dicyclohexylamin, Triethanolamin, Ethylendiamin, Tris(hydroxymethyl)-aminome-than, Benzyltrimethylammoniumhydroxyd und dergleichen ab.

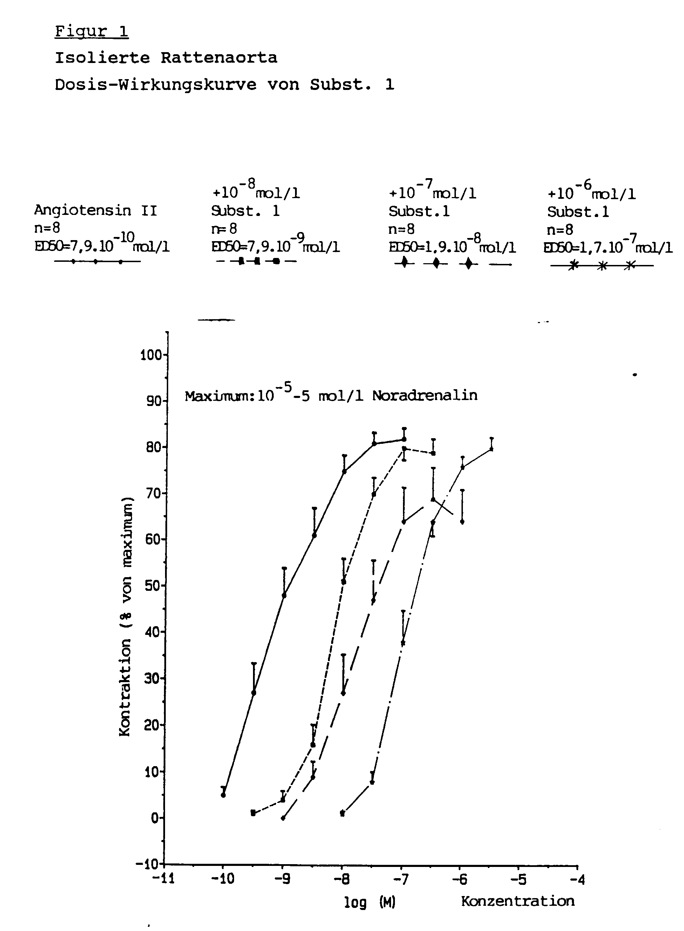

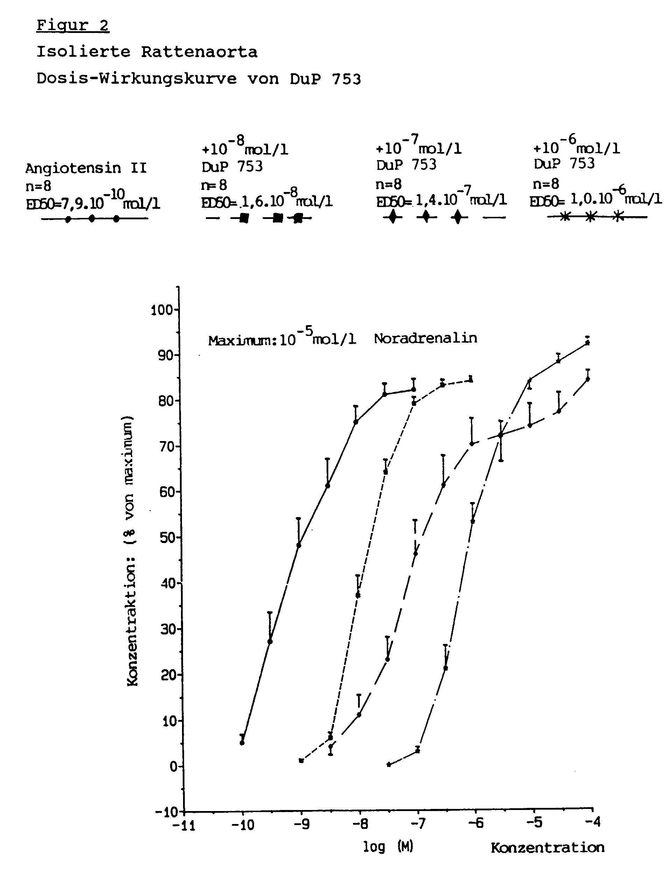

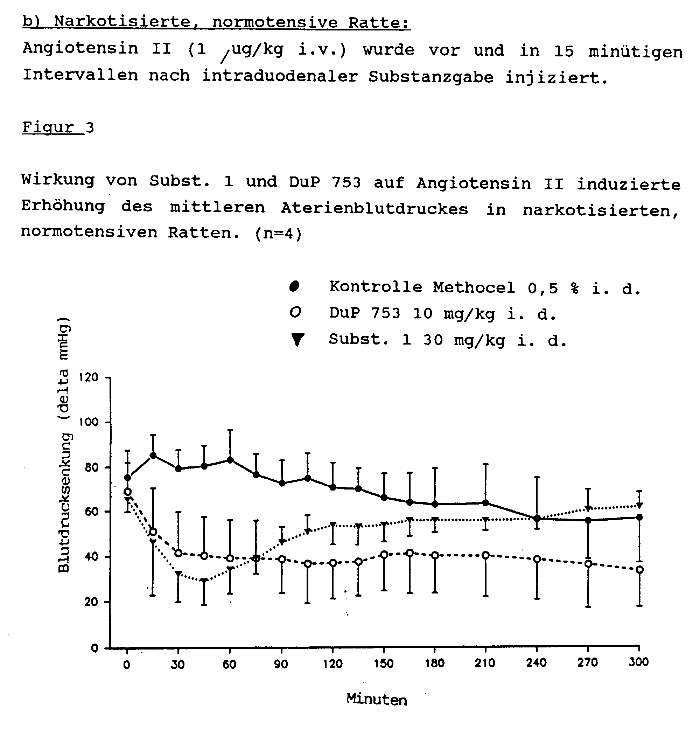

Die neuen Verbindungen der Formel I und ihre Salze sind oral wirksam und unterdrücken die vasokonstriktive und blutdrucksteigernde Wirkung von Angiotensin II.
In Tiermodellen zeigen die Verbindungen hervorragende blutdrucksenkende Wirkung.
So wird zum Beispiel (Fig. 1) die Dosis-Wirkungskurve von Angiotensin II dosisbezogen und kompetitiv nach rechts verschoben. Bei Vergleichsversuchen mit DuP 753 (Fig. 2) zeigt sich, daß diese Substanz bei invitro Versuchen zwar auch kompetitiv wirkt, bei invivo Versuchen (Fig. 3) ist jedoch die Umwandlung in einen aktiven nicht kompetitiven Metaboliten in der im Vergleich zu den erfingunsgemäßen Substanzen längerer Wirkungsdauer erkennbar. Versuche von Wong et al., 1990, JPET, Vol.255, Seiten 211 - 217 haben gezeigt, daß dieser aktive Metabolit EXP 3174, ein nicht kompetitiver irreversibler Angiotensin II Rezeptor-Antagonist, für die lange Wirkungsdauer verantwortlich ist.
Die erfingunsgemäßen Substanzen werden hingegen reversibel an den Rezeptor gebunden, so daß dieser nicht verändert oder zerstört wird. Diese reversible Bindung äußert sich in der kürzeren Wirkungsdauer, wodurch auch gezeigt wird, daß diese Substanzen ohne einen aktiven nicht kompetitiven Metaboliten wirken.
Aufgrund dieser pharmakologischen Eigenschaften können die neuen Verbindungen allein oder in Mischung mit anderen Wirksubstanzen in Form üblicher galenischer Zubereitung als Heilmittel zur Behandlung von Bluthochdruck und anderen cardiovaskulären Erkrankungen Verwendung finden.

Die Erfindung bezieht sich weiterhin auf Arzneimittel, die z. B. in Form pharmazeutischer Präparate Verwendung finden, welche die erfindungsgemäßen Verbindungen der Formel I oder ihre Salze in Mischung mit einem oder mehreren für die enterale oder parenterale Applikation geeigneten, pharmazeutischen, organischen oder anorganischen Trägermaterial, beispielsweise Wasser, Gelatine, Gummi arabicum, Milchzucker, Stärke, Magnesiumstearat, Talk, pflanzliche Öle, Polyalkylenglykole, Vaseline oder dergleichen enthalten.

Die pharmazeutischen Präparate können in fester Form z. B. als Tabletten, Filmtabletten, Dragees, Suppositorien, Kapseln, Mikrokapseln oder in flüssiger Form z. B. als Lösungen, Injektionslösungen, Suspensionen oder Emulsionen oder in Zusammensetzungen mit verzögerter Freigabe des Wirksktoffes vorliegen. Gegebenenfalls sind sie sterilisiert und/oder enthalten Hilfsstoffe, wie Konservierungs-, Stabilisierungs- oder Emulgiermittel, Salze zur Veränderung des osmotischen Druckes oder Puffer.

Insbesondere können pharmazeutische Präparate die erfindungsgemäßen Verbindungen in Kombination mit anderen therapeutisch wertvollen Stoffen enthalten. Mit diesen können die erfindungsgemäßen Verbindungen zusammen mit den oben angegebenen Hilfs- und/oder Trägerstoffen zu Kombinationspräparaten formuliert werden.
Eine geeignete Dosis zur Verabreichung der neuen Verbindungen beträgt etwa 4 - 200 mg/kg pro Tag, bevorzugte Tagesdosis/Patient beträgt etwa 60 - 200 mg, jedoch kommen, je nach dem Zustand des zu behandelnden Patienten, auch andere Dosen in Frage. Die neuen Verbindungen können in mehreren Dosen und auf oralem Weg verabreicht werden.

Die neuen Verbindungen können in den erfindungsgemäßen pharmazeutischen Zusammensetzungen in einem Anteil von etwa 4 - 200 mg pro Tablette,vorzugsweise etwa 20 - 50 mg pro Tablette, vorhanden sein, wobei der Rest ein pharmazeutisch annehmbarer Füllstoff ist.

### Beispiel 1:

### 5-(4'-(2-Butyl-4-chlor-5-(3-methyl-1,2,4-oxadiazol-5-yl)1-imidazolylmethyl)biphenyl-2-yl)1-triphenylmethyl-1H-tetrazol

Zu einer Lösung von 0,28 g (0,0038 Mol) N-Hydroxy-ethan-imidamid in 20 ml abs. THF werden 0,09 g (0,0038 Mol) Natriumhydrid gegeben und diese Suspension eine Stunde über Molsieb unter Stickstoffatmosphäre auf 60°C erhitzt. Das Gemisch wird auf 0°C gekühlt und eine Lösung von 2,20 g (0,0032 Mol) 1-((2'-(N-triphenylmethyl-tetrazol-5-yl)-biphenyl-4-yl)methyl)-2-butyl-4-chlor-1H-imidazol-5-carbonsäuremethylester in 45 ml abs. THF rasch zugetropft. Die Suspension wird auf Raumtemperatur erwärmt und 18 Stunden gerührt. Das Reaktionsgemisch wird filtriert und das Filtrat im Vakuum eingedampft. Der Rückstand wird zwischen 10 ml Wasser und 10 ml Essigsäureethylester verteilt, die Phasen getrennt und die wäßrige Phase mit 3 x 20 ml Essigsäureethylester extrahiert. Die vereinten organischen Phasen werden über Natriumsulfat getrocknet, filtriert und das Lösungsmittel abgezogen. Die Reinigung des Produkts erfolgt durch Säulenchromatographie. (KG60; 30 g; LM: Tol:EE = 10:1). Das Produkt wird durch Digerieren in Ether zur Kristallisation gebracht.
Ausbeute: 1,00 g farblose Kristalle (43 % d. Th.)
Fp: 175 - 177°C (Aceton)
DC: LM: Tol : EE = 10 : 1 R_{f} = =0,4

| Mikroelementaranalyse: | | | |
|---|---|---|---|
| C₄₃H₃₇N₈ClO | MG = 717,28 | | |
| | C | H | N |
| berechnet | 72,01 | 5,20 | 15,62 |
| gefunden | 71,80 | 5,37 | 15,68 |

¹H-NMR (CDCl₃)
delta (ppm): 7,93 - 7,87 (m; 1H; Bz-H₃'); 7,63 - 7,47 (m; 2H; Bz-H₅',-H₆'); 7,41 - 7,26 (m; 1H; Bz-H₄'); 7,39 - 7,28 (m; 10H; Bz-H); 7,11; 7,07; 6,78; 6,72 (A₂B₂; 4H; Bz-H₂,-H₆ u. Bz-H₃,-H₅,); 6,99 - 6,89 (m; 6H; Bz-H); 5,67 (s; 2H; Im-CH₂-Bz); 2,53 (t; 2H; Bu₁-CH₂); 2,35 (s;3H; -CH₃); 1,63 (m; 2H; Bu₂-CH₂); 1,22 (m; 2H; Bu₃-CH₂); 0,84 (t; 3H; Bu-CH₃)

¹³C-NMR (CDCl₃)
delta (ppm): 166,68; 163,92; 153,06; 141,21; 140,94; 134,16; 130,72; 130,21; 129,98; 129,83; 128,26; 127,61; 126,22; 125,53; 113,83; 82,87; 48,49; 29,29; 26,94; 22,31; 13,70; 11,51

### 5-(4'-(2-Butyl-4-chlor-5-(3-methyl-1,2,4-oxadiazol-5-yl)1-imidazolylmethyl)biphenyl-2-yl)1H-tetrazol.

0,40 g (0,0006 Mol) 5-(4'-(2-Butyl-4-chlor-5-(3-methyl-1,2,4-oxadiazol-5-yl)l-imidazolyl-methyl)biphenyl-2-yl)1-triphenylmethyl-1H-tetrazol werden in 12 ml Methanol suspendiert und zwei Stunden zum Sieden erhitzt. Das Lösungsmittel wird abgezogen, der Rückstand mit 3 ml siedendem Diethylether in der Hitze digeriert und die erhaltenen Kristalle abfiltriert. Das Rohprodukt wird aus Essigsäureethylester/Aktivkohle umkristallisiert.
Ausbeute: 0,10 g farblose Kristalle (35 % d. Th.)
Fp: 178 - 180°C (EE)
DC: LM: Bz : Dx : AcOH = 8 : 1 : 1 R_{f} = 0,6

| Mikroelementaranalyse: | | | |
|---|---|---|---|
| C₂₄H₂₃N₈ClO | MG = 474,96 | | |
| | C | H | N |
| berechnet | 60,69 | 4,88 | 23,59 |
| gefunden | 60,51 | 4,94 | 23,29 |

¹H-NMR (CDCl₃)
delta (ppm): 7,93 - 7,87 (m; lH; Bz-H₃'); 7,63 - 7,47 (m; 2H; Bz-H₅',-H₆') ; 7,41 - 7,26 (m; lH; Bz-H₄') ; 7,11; 7,07; 6,92; 6,87 (A₂B₂; 4H; Bz-H₂, -H₆ u. Bz-H₃,-H₅,); 5,67 (s; 2H; Im-CH₂-Bz); 2,53 (t; 2H; Bu₁-CH₂); 2,35 (s; 3H; -CH₃); 1,63 (m; 2H; Bu₂-CH₂); 1,22 (m; 2H; Bu₃-CH₂); 0,84 (t; 3H; Bu-CH₃)

¹³C-NMR (CDCl₃)
delta (ppm): 166,94; 166,36; 153,01; 140,58; 139,30; 135,51; 135,16; 131,38; 130,79; 130,66; 129,60; 128,42; 126,25; 122,89; 113,54; 48,59; 29,56; 26,74; 22,27; 13,66; 11,54

### Beispiel 2:

### Angiotensin II-Rezeptor-antagonistische Wirkungen von der Substanz aus Beispiel 1 (Subst. 1) im Vergleich zur Referenzsubstanz DuP 753

### a) Isolierte Rattenaorta:

Kumulative Dosis-Wirkungskurven von Angiotensin II (10⁻¹⁰-10⁻⁷ mol/l) wurden mit und ohne Vorbehandlung mit der Substanz aus Beispiel 1) oder DuP 753 (je 10⁻⁸, 10⁻⁷, 10⁻⁶ mol/l) isometrisch durchgeführt und als Prozent der maximalen Kontraktion durch Noradrenalin (10⁻⁵ mol/l) dargestellt. ED₅₀-Werte und pA₂-Werte wurden rechnerisch ermittelt.

## Patentansprüche

1. Tetrazolderivate der Formel 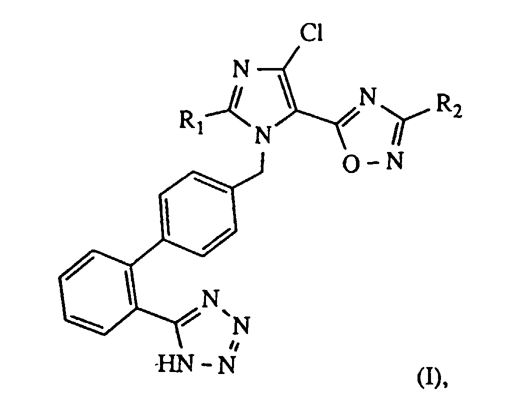 in der R₁ einen gesättigten oder ungesättigten, geradkettigen oder verzweigten (C₁-C₆) Alkylrest und R₂ Methyl oder Ethyl bedeuten, sowie ihre pharmazeutisch verwendbaren Salze.

2. Verbindungen der in Anspruch 1 definierten Formel I, worin R₁ Butyl bedeutet.

3. 5-(4'-(2-Butyl-4-chlor-5-(3-methyl-1,2,4-oxadiazol-5-yl)1-imidazolylmethyl)biphenyl-2-yl)-1H-tetrazol gemäß Anspruch 1.

4. Verfahren zur Herstellung von Verbindungen der Formel 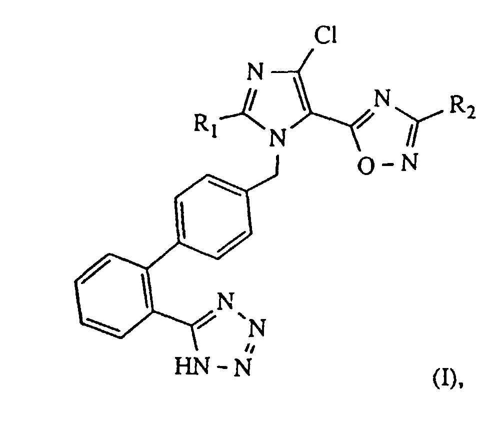 in der R₁ einen gesättigten oder ungesättigten, geradkettigen oder verzweigten (C₁ - C₆) Alkylrest und R₂ Methyl oder Ethyl bedeuten, sowie ihre pharmazeutisch verwendbaren Salze,
dadurch gekennzeichnet, daß eine Verbindung der Formel 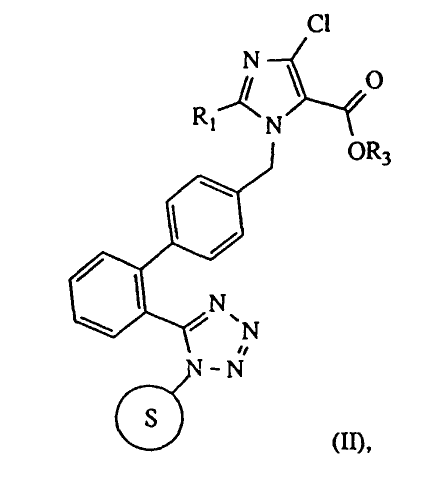 in der R₁ die obige Bedeutung hat und R₃ Methyl oder Ethyl und S eine Schutzgruppe bedeuten, mit einer Verbindung der Formel 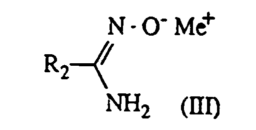 in der R₂ Methyl oder Ethyl und Me Natrium oder Kalium bedeuten, in einem unter Reaktionsbedingungen inerten organischen Verdünnungsmittel unter Inertgasatmosphäre zu Verbindungen der Formel 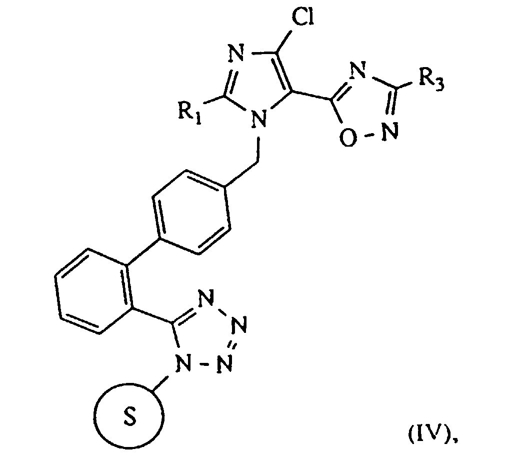 in der R₁ und R₂ die obige Bedeutung haben, umgesetzt und anschließend die Schutzgruppe entfernt wird.

5. Verfahren nach Anspruch 4, dadurch gekennzeichnet, daß die Schutzgruppe durch Behandeln mit starken Säuren oder durch Erhitzen mit niederen aliphatischen Alkoholen abgespalten wird.

6. Verfahren nach Anspruch 4, dadurch gekennzeichnet, daß als Schutzgruppe die Triphenylmethylgruppe verwendet wird.

7. Pharmazeutische Präparate, enthaltend Verbindungen der Formel I nach Anspruch 1 sowie deren Salze in Kombination mit üblichen galenischen Hilfs- und/oder Trägerstoffen.

8. Pharmazeutische Präparate, enthaltend Verbindungen der Formel I nach Anspruch 1 sowie deren Salze in Kombination mit anderen therapeutisch wertvollen Wirkstoffen sowie üblichen galenischen Hilfs- und/oder Trägerstoffen.

9. Verbindungen nach Anspruch 1 und deren Salze zur Verwendung als Wirkstoffe für Arzneimittel zur Behandlung von Krankheiten, die durch Hemmung von Angiotensin II gelindert oder geheilt werden können.

10. Verwendung von Verbindungen nach Anspruch 1 als blutdrucksenkende Arzneimittel.

## Claims

1. Tetrazole derivatives of the formula 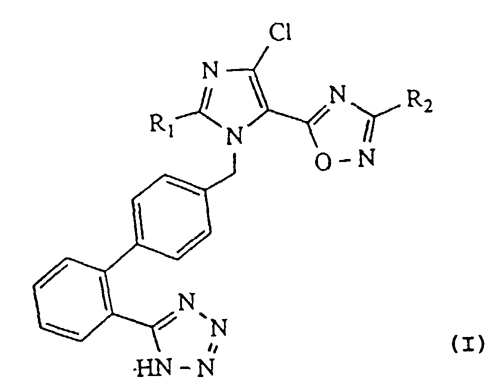 in which R₁ denotes a saturated or unsaturated, straight-chain or branched (C₁-C₆) alkyl radical and R₂ denotes methyl or ethyl, and their pharmaceutically usable salts.

2. Compounds of the formula I defined in Claim 1, wherein R₁ denotes butyl.

3. 5-(4'-(2-Butyl-4-chloro-5-(3-methyl-1,2,4-oxadiazol-5-yl)-1-imidazolylmethyl)biphenyl-2-yl)-1H-tetrazole according to Claim 1.

4. Process for the preparation of compounds of the formula 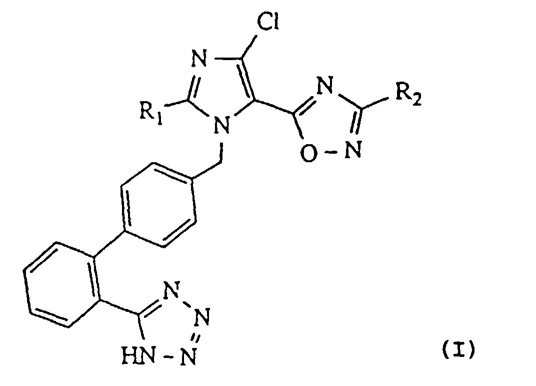 in which R₁ denotes a saturated or unsaturated, straight-chain or branched (C₁ - C₆) alkyl radical and R₂ denotes methyl or ethyl, and their pharmaceutically usable salts, characterized in that a compound of the formula 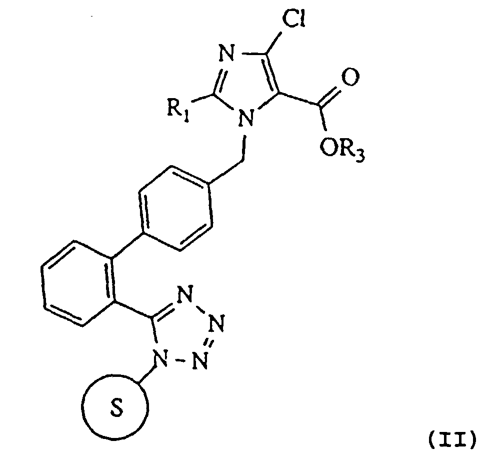 in which R₁ has the above meaning, R₃ denotes methyl or ethyl and S denotes a protective group, is reacted with a compound of the formula 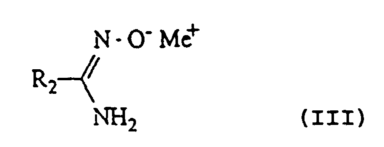 in which R₂ denotes methyl or ethyl and Me denotes sodium or potassium, in an organic diluent which is inert under the reaction conditions, under an inert gas atmosphere, to give compounds of the formula 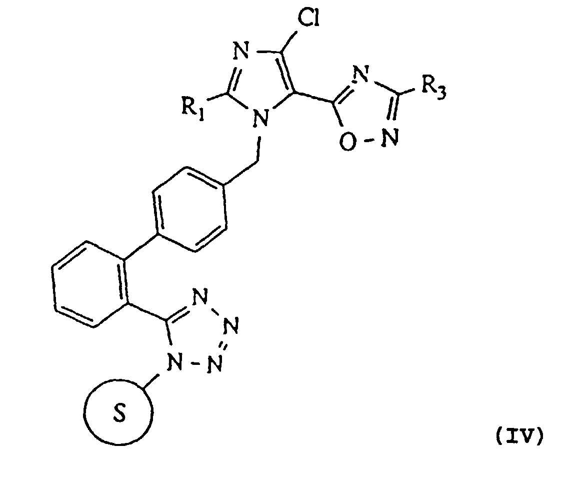 in which R₁ and R₂ have the above meaning, and the protective group is then removed.

5. Process according to Claim 4, characterized in that the protective group is split off by treatment with strong acids or by heating with lower aliphatic alcohols.

6. Process according to Claim 4, characterized in that the triphenylmethyl group is used as the protective group.

7. Pharmaceutical preparations containing compounds of the formula I according to Claim 1 and salts thereof, in combination with customary pharmaceutical auxiliaries and/or excipients.

8. Pharmaceutical preparations containing compounds of the formula I according to Claim 1 and salts thereof, in combination with other therapeutically useful active compounds and customary pharmaceutical auxiliaries and/or excipients.

9. Compounds according to Claim 1 and salts thereof for use as active compounds for medicaments for the treatment of diseases which can be alleviated or cured by inhibition of angiotensin II.

10. Use of compounds according to Claim 1 as antihypertensive medicaments.

## Revendications

1. Dérivés tétrazoliques de formule 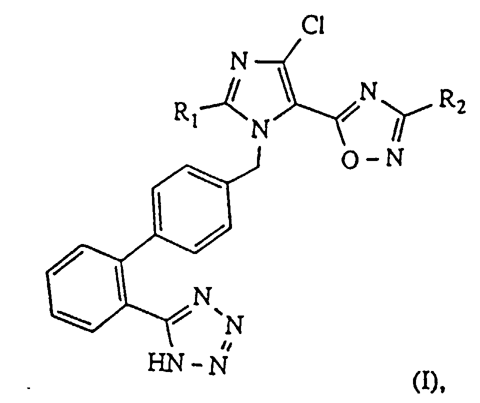 où R₁ signifie un reste alkyle en C₁-C₆, saturé ou insaturé, linéaire ou ramifié, et R₂ signifie un reste méthyle ou éthyle, ainsi que leurs sels pharmaceutiquement utilisables.

2. Composés répondant à la formule I définie dans la revendication 1, où R₁ signifie le reste butyle.

3. 5-(4'-(2-butyl-4-chloro-5-(3-méthyl-1,2,4-oxadiazol-5-yl)-1-imidazolylméthyl)biphényl-2-yl)-1H-tétrazole selon la revendication 1.

4. Procédé de préparation de composés répondant à la formule 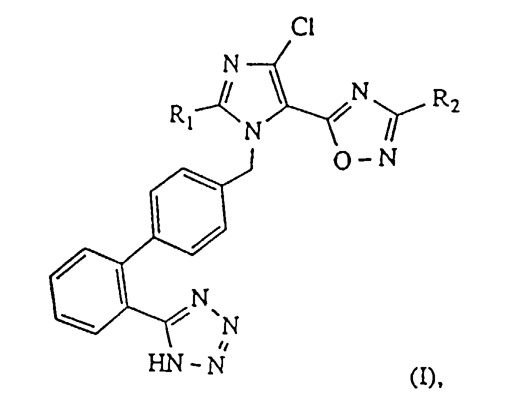 où R₁ signifie un reste alkyle en C₁-C₆, saturé ou insaturé, linéaire ou ramifié, et R₂ signifie un reste méthyle ou éthyle, ainsi que leurs sels pharmaceutiquement utilisables,
caractérisé en ce qu'on fait réagir un composé de formule 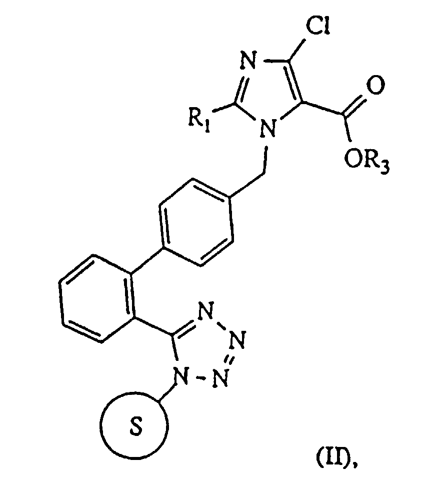 où R₁ a la signification indiquée ci-dessus, R₃ signifie le reste méthyle ou éthyle, et S signifie un groupe protecteur, avec un composé de formule 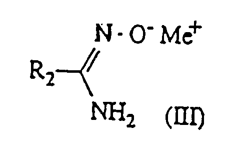 où R₂ signifie le reste méthyle ou éthyle, et Me signifie le sodium ou le potassium, dans un diluant organique inerte dans les conditions de réaction, dans une atmosphère de gaz inerte, pour obtenir des composés de formule 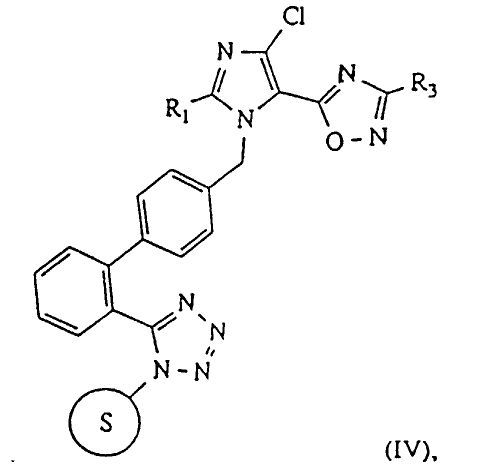 où R₁ et R₂ ont les significations précédentes, et ensuite, on élimine le groupe protecteur.

5. Procédé selon la revendication 4, caractérisé en ce que le groupe protecteur est séparé par traitement avec des acides forts ou par chauffage avec des alcools aliphatiques inférieurs.

6. Procédé selon la revendication 4, caractérisé en ce qu'on utilise le groupe triphénylméthyle comme groupe protecteur.

7. Préparations pharmaceutiques contenant des composés de formule I selon la revendication 1, ainsi que leurs sels, combinés avec des adjuvants et/ou des matériaux de support usuels.

8. Préparations pharmaceutiques contenant des composés de formule I selon la revendication 1, ainsi que leurs sels, combinés avec d'autres principes actifs précieux du point de vue thérapeutique, ainsi que des adjuvants et/ou des matériaux de support usuels.

9. Composés selon la revendication 1 et leurs sels, destinés à être utilisés comme des principes actifs pour des médicaments servant au traitement de maladies qu'on peut atténuer ou guérir par inhibition de l'angiotensine II.

10. Utilisation des composés selon la revendication 1 comme des médicaments hypotenseurs.
